# EUROPEAN PATENT SPECIFICATION

(11) **EP 2 883 962 B1**
(45) Date of publication and mention of the grant of the patent: **13.09.2017**
(21) Application number: 13197367.9
(22) Date of filing: 16.12.2013
(51) Int. Cl.: C12Q 1/68

(54) **High levels of EMT-TFs for the diagnosis of cancer, in particular of colorectal (CRC) and pancreatic (PC) cancer**
Hohes Maß an EMT-TF zur Diagnose von Krebs, insbesondere von kolorektalem Krebs (KRK) und Pankreaskrebs (PK)
Niveaux élevés de EMT-TFs pour le diagnostic du cancer, en particulier deu cancer colorectal (CRC) et pancréatique (PC)

(43) Date of publication of application: 17.06.2015
(73) Proprietor: Humanitas Mirasole S.p.A., 20089 Rozzano (Milano) (IT)
(72) Inventor: Laghi, Luigi, 20089 Rozzano (MI) (IT); Celesti, Giuseppe, 20089 Rozzano (MI) (IT)
(74) Representative: Capasso, Olga

(56) References cited:
- WO-A1-2012/149014
- WO-A2-2012/103025
- GIUSEPPE CELESTI ET AL: "Presence of Twist1-Positive Neoplastic Cells in the Stroma of Chromosome-Unstable Colorectal Tumors", GASTROENTEROLOGY, vol. 145, no. 3, 1 September 2013 (2013-09-01), pages 647-657.e15, XP055109802, ISSN: 0016-5085, DOI: 10.1053/j.gastro.2013.05.011
- M. J. LARRIBA ET AL: "Snail2 cooperates with Snail1 in the repression of vitamin D receptor in colon cancer", CARCINOGENESIS, vol. 30, no. 8, 5 June 2009 (2009-06-05), pages 1459-1468, XP055110456, ISSN: 0143-3334, DOI: 10.1093/carcin/bgp140
- KENOKI OHUCHIDA ET AL: "Twist, a novel oncogene, is upregulated in pancreatic cancer: Clinical implication of Twist expression in pancreatic juice", INTERNATIONAL JOURNAL OF CANCER, vol. 120, no. 8, 15 April 2007 (2007-04-15), pages 1634-1640, XP055110763, ISSN: 0020-7136, DOI: 10.1002/ijc.22295
- HUNG PHAM ET AL: "Loss of 15-Hydroxyprostaglandin Dehydrogenase Increases Prostaglandin E2 in Pancreatic Tumors", PANCREAS, vol. 39, no. 3, 1 April 2010 (2010-04-01), pages 332-339, XP055110768, ISSN: 0885-3177, DOI: 10.1097/MPA.0b013e3181baecbe
- Y IMAMICHI ET AL: "Collagen type I-induced Smad-interacting protein 1 expression downregulates E-cadherin in pancreatic cancer", ONCOGENE, vol. 26, no. 16, 9 October 2006 (2006-10-09), pages 2381-2385, XP055110206, ISSN: 0950-9232, DOI: 10.1038/sj.onc.1210012
- Anonymous: "宝生?工程(?&#x8FD E;)??公司", , 26 December 2010 (2010-12-26), XP055110594, Retrieved from the Internet: URL:http://web.archive.org/web/20101226043 052/http://www.takara.com.cn/?action=Page& Plat=pdetail&newsid=730&subclass=1 [retrieved on 2014-03-28]

## Description

### Field of the invention

The present invention refers to the field of molecular markers for disease diagnosis, preferably for cancer diagnosis. The present invention is aimed at detecting and measuring mRNA levels of genes involved in epithelial to mesenchymal transition in biological samples, i.e. in peripheral blood samples of tumor patients, to determine the presence of disease, its progression and risk of recurrence.

### Background

Circulating tumor cells (CTCs) are an heterogeneous population of cancer cells circulating in the peripheral blood that have been shed from either a primary tumor or its metastasis. Currently, the raw number and corresponding phenotype of CTCs in the whole blood of cancer patients has clinical relevance with respect to patients prognosis. CTC pool includes cells with epithelial, mesenchymal and stemness-like features. It has been shown that cancer intravasation in humans (Min Yu et al., Science, 2013, 339, 580) and animals is coupled with epithelial to mesenchymal transition (EMT), a process driven by transcription factors (TFs; like but not limited to *TWIST1, ZEB2, SLUG, SNAIL*, etc) and likely reversible (mesenchymal-to-epithelial transition, MET) at metastatic niches. Therefore, the detection of EMT cells in human blood is expected to provide diagnostic and prognostic clues.

Celesti et al. (Gastroenterology 2013, 145, 647) showed that levels of *TWIST1* mRNA were statistically (median value) significantly higher in blood samples from patients with Colo-Rectal Cancer (CRC) than from controls. Unfortunately the distribution of *TWIST1* levels in patients and controls partially overlaps, rendering the measurement of *TWIST1* mRNA alone not reliable and suitable for diagnosis.

M. J. Larriba et al. (Carcinogenesis, vol. 30, no. 8, 2009) refers to SLUG overexpression in colorectal cancer, but is silent about TWIST1, SLUG and ZEB2 expression levels in blood.

Kenoki Ohuchida et al. (International Journal of cancer, vol. 120, no. 8, 2007) refers to TWIST1 mRNA overexpression in pancreatic cancer, but is silent about TWIST1, SLUG and ZEB2 expression levels in blood.

Hung Pham et al. (Pancreas, vol. 39, no. 3, 2010) refers to SLUG mRNA overexpression in pancreatic cancer, but is silent about TWIST1, SLUG and ZEB2 expression levels in blood.

Y. Imamichi et al., (Oncogene, vol. 26, no. 16, 2006) refers to ZEB2 mRNA overexpression in pancreatic cancer, but is silent about TWIST1, SLUG and ZEB2 expression levels in blood.

WO2012/103025 refers to a method for assessing cancer progression in a patient suffering from cancer, comprising determining RNA expression level of a cancer specific gene in a sample enriched in circulating tumor cells (CTCs), wherein the EMT marker is selected from the group consisting of: N-cadherin, vimentin, B-catenin (nuclear localized), Snail-1, Snail-2 (Slug), Twist, EF1/ZEB1, SIP1/ZEB2, and E47.

### Description of the invention

Conventional, FDA-approved methods (i.e. Cell Search" (Veridex), "Adna Test Breast Cancer Select" and "Adna test Breast Cancer Detect" (AdnaGen AG), "Biocept Onco CEE" (Biocept Laboratories) detect epithelial antigens expressed, among others, by CTCs. Hence the diagnostic and prognostic value of these tests is limited.

The authors show quantitative RT-PCR assay specific for epithelial-mesenchymal transition (EMT) genes present in circulating cells of tumor patients. Differently from what reported by prior art, no prior separation of circulating cell populations is needed prior to mRNA extraction. This approach allows detecting increased levels of circulating EMT-TF transcripts, irrespective of any antigenic or phenotypic cell feature.

Authors found that high levels of both *TWIST1* and *SLUG* mRNAs are present in blood cells of CRC patients, whereas high levels of *TWIST1*, *SLUG*, and *ZEB2* mRNAs are present in blood cells of pancreatic cancer (PC) patients, thus allowing a more reliable and robust diagnosis.

Therefore it is an object of the instant invention a method for determining if a subject is affected by a cancer comprising the step of assaying a biological sample from said subject for the presence of a panel of mRNAs encoding for transcription factors involved in epithelial to mesenchymal transition, wherein :
a) said panel comprises at least TWIST1, SLUG and ZEB2 mRNAs,
b) the increase of mRNA levels of both TWIST1 and SLUG genes but not of ZEB2 gene with respect to control samples is indicative of a colorectal cancer,
c) the increase of mRNA levels of all of TWIST1, SLUG and ZEB2 genes with respect to control samples is indicative of a pancreatic cancer,
wherein said biological sample is a blood cell sample.

In a preferred embodiment of the invention the subject is not administered an antitumor treatment (e.g., neo-adjuvant therapy).

Advantageously the blood cell sample is a Circulating Tumor Cells (CTCs) enriched sample, more preferably a Peripheral Blood Mononuclear Cell (PBMC) sample.

Authors selected appropriate cut-offs to as follows:
- TWIST1 mRNA : 9.30E-09 if the subject is affected by a colorectal cancer, or 2.57E-8 if the subject is affected by a pancreatic cancer;
- SLUG mRNA: 1.83E-10 if the subject is affected by a colorectal cancer, or 5.70E-9 if the subject is affected by a pancreatic cancer;
- ZEB2 : 4.08E-05 if the subject is affected by a colorectal cancer, as indication upon cancer treatment of partial or full remission of colorectal cancer, and/or colorectal cancer stage variation and/or treatment responsiveness, or 26.84E-6 if the subject is affected by pancreatic cancer.

In a preferred embodiment the levels of mRNAs are obtained by RT-PCR. Any other method to detect specific mRNA in a biological sample known to the expert in the art are within the scope of the instant invention. Illustrative examples are: PCR amplification methods (QX200™ Droplet Digital™ PCR System, TaqMan Probes), other amplification methods; up to single cell gene expression analysis (miRGE - nCounter^{®}).

The method of the invention is able to detect EMT transcripts in blood samples of humans or animals; in a particular aspect the method comprises the steps of :
a) Ficoll gradient density separation of Peripheral Blood Mononuclear Cells (PBMC),
b) detection of the expression levels of EMT genes using RT-PCR,
c) comparison of the level of the EMT genes from the patient to normal control levels,
d) diagnosing the patient as having a specific tumor if the detected levels of the EMT genes are statistically significantly different (higher or lower than a predetermined cut off value depending on the selected gene) than the control level.

It is a further objet of the invention the use of a quantitative RT-PCR kit for working the method above disclosed including:
- retrotranscribing means to get specific cDNAs;
- specific amplification probes for amplifying specific cDNAs;
- detecting means to detect and measure the level of amplified specific cDNAs.

In a preferred embodiment of the use of the quantitative RT-PCR kit of the invention, amplification probes are able to amplify:
- the nt. 781-835 region of *TWIST1,* Acc. No.: NM_000474.3, (SEQ ID No. 1);
- the nt. 711-819 region of *SLUG,* Acc. No.: NM_003068.3, (SEQ ID No. 2);
- the nt. 1262-1352 region of *ZEB2* Acc. No.: NM_014795.3, (SEQ ID No. 3);
- the nt. 174-244 region of *SNAIL,* Acc. No.: NM_005985.3, (SEQ ID No. 4).

In a further preferred embodiment of the use of the quantitative RT-PCR kit of the invention, amplification probes have essentially the sequences:
TWIST1-Forward AGCAAGATTCAGACCCTCAAGCT (SEQ ID No. 8);
TWIST1-Reverse CCTGGTAGAGGAAGTCGATGTACCT (SEQ ID No. 9);
SLUG-Forward TGTTTGCAAGATCTGCGGC (SEQ ID No. 10);
SLUG-Reverse TGCAGTCAGGGCAAGAAAAA (SEQ ID No. 11);
ZEB2-Forward GCTACACGTTTGCCTACCGC (SEQ ID No. 12);
ZEB2-Reverse CGATTACCTGCTCCTTGGGTT (SEQ ID No. 13).

### Detailed description of the invention

The invention shall be described with reference to non-limitative examples.

### Figure legends

**Figure 1****.** mRNA levels of TWIST1 (A),SLUG (B),ZEB2 (C), and SNAIL (D) in blood of 26 CRC patients as compared to 5 Miscellanea patients and to 16 healthy controls.
**Figure 2****.** ROC curve for TWIST1 (A),SLUG (B) mRNA levels in 26 CRC patients as compared to 16 healthy controls.
**Figure 3****.** ROC curve for ZEB2 mRNA levels in 26 CRC patients as compared with 5 Miscellanea patients.
**Figure 4****.** mRNA levels of TWIST1 (A),SLUG (B),ZEB2 (C), and SNAIL (D) in blood of 10 PC patients as compared to 16 healthy controls.
**Figure 5****.** ROC curve for TWIST1 (A),SLUG (B) and ZEB2 (C) mRNA levels in 10 PC patients as compared to 16 healthy controls.
**Figure 6****.** Comparison of blood levels of ZEB2 mRNA in CRC and PC (A).
ROC curve for ZEB2 (B) mRNA levels in 26 patients with a colorectal cancer as compared to 10 patients with PC.

### Materials and Methods

### Quantification of Gene Transcripts in unselected blood samples

Peripheral blood (6mL) was collected in anticoagulants (EDTA, sodium citrate, heparin)-coated vacutainer and stored at 4°C. Peripheral blood was processed within 4 hours of collection and Ficoll-Paque Plus (GE Healthcare, Life Science) gradient separated according to the manufacturer's instructions. Briefly, 15 mL of Ficoll-Paque was added to a centrifuge tube and diluted PBS (6 mL+29 mL of balanced salt solution) was I carefully layered on Ficoll-plaque. The unmixed solution was centrifuged (400 g for 40 minutes at 20°C). Using a clean Pasteur pipette the upper layer was removed leaving untouched the lymphocyte layer at the interface. Next, by a new Pasteur pipette the PBMC layer containing the circulating tumor cells was transferred to a clean centrifuge tube and washed with at least 3 volumes (18 ml) of balanced salt solution. Once re-suspended, cells were centrifuged (300 g for 20 minutes at 20°C), and the supernatant was then removed. After adding 50 mL of balanced salt solution cells pellet was finally centrifuged (200 g for 20 minutes at 20°C). The supernatant was again removed and cells lysed with Qiagen lysis buffer plus β-Mercaptoethanol (1:100). Total RNA was then isolated using Qiagen Rneasy-Mini kit according to manufacturer's instructions. Thereafter, total RNA re-suspended in 60 µL of dethylphyrocarbonate-treated water (DEPC-water) was treated by DNAse (Ambion, Life Science) to minimize the contamination by genomic DNA.

All RNA preparation and handling steps took place in a laminar flow hood, under RNA-free conditions. RNA concentration was determined by absorbance reading at 260 nm using nanodrop.

Two µg of treated RNA was reverse transcripted to cDNA using High Capacity cDNA Reverse Transcription Kit (Applied Biosystem, Life Science). Synthesized cDNA was subjected to RT-PCR to detect and quantify EMT-transcription factor mRNA levels:
*TWIST1:* NCBI Acc. No. NM_000474.3 (SEQ ID No.1),
*SLUG or SNAI2*: NCBI Acc. No. NM_003068.3 (SEQ ID No.2),
*ZEB2 or SIP1*: NCBI Acc. No. NM_014795.3 (SEQ ID No.3),
*SNAIL*: NCBI Acc. No. NM_005985.3 (SEQ ID No.4).

In brief, 1 µl of cDNA (40ng) was placed in 20 µL of reaction volume containing 12 µl of Fast SyberGreen Master Mix, 3 µl of housekeeping and target Forward and Reverse Primers mixed at 5µM and 4 µl of DEPC-water. Specific primer sequences were:
18s-Forward CGC CGC TAG AGG TGA AAT TCT (SEQ ID No. 6),
18s-Reverse CTT TCG CTC TGG TCC GTC TT (SEQ ID No. 7);
to amplify the nt. 1049-1100 region of 18s, Acc. No.: M10098.1, NCBI, (SEQ ID No. 5), as control;
TWIST1-Forward AGC AAG ATT CAG ACC CTC AAG CT (SEQ ID No. 8);
TWIST1-Reverse CCT GGT AGA GGA AGT CGA TGT ACC T (SEQ ID No. 9);
to amplify the nt. 781-835 region of *TWIST1,* Acc. No.: NM_000474.3, (SEQ ID No. 1);
SLUG-Forward TGT TTG CAA GAT CTG CGG C (SEQ ID No. 10);
SLUG-Reverse TGC AGT CAG GGC AAG AAA AA (SEQ ID No. 11);
to amplify the nt. 711-819 region of *SLUG,* Acc. No.: NM_003068.3, (SEQ ID No. 2);
ZEB2-Forward GCT ACA CGT TTG CCT ACC GC (SEQ ID No. 12);
ZEB2-Reverse CGA TTA CCT GCT CCT TGG GTT (SEQ ID No. 13);
to amplify the nt. 1262-1352 region of *ZEB2* Acc. No.: NM_014795.3, (SEQ ID No. 3);
SNAIL-Forward CTT CCA GCA GCC CTA CGA C, (SEQ ID No. 14);
SNAIL Reverse CGG TGG GGT TGA GGA TCT(SEQ ID No. 15);
to amplify the nt. 174-244 region of *SNAIL,* Acc. No.: NM_005985.3, (SEQ ID No. 4).

Amplification was performed in an ABI 7900 HT Real Time PCR system (Applied Biosystem) using the program: 95°C for 10 minutes; 40 cycles of 95°C for 15s and 60°C for 60s. All samples were analyzed in triplicate. Quantification of target genes and internal reference gene 18s was performed using the fluorescence emission of SyberGreen. Proper DNA contamination was assessed by performing PCR on the no-reverse transcribed portion of each sample. For all samples fluorescence was detected after 0-40 cycles for the control and marker genes in a single reaction, which allow for the deduction of the cycles at threshold (CT) value for each product. The CT value was considered as the PCR cycle at which a significant increase in fluorescence is detected due to the exponential accumulation of double-strand PCR products. Expression of the target genes was normalized on the expression of the housekeeping gene, 18s to obtain the absolute quantification (2^{-ΔCT}), while the relative quantification was calculated by 2^{-ΔΔCT} method to compare the transcript levels of controls versus patients (Livak Kj, Methods, 2001;25(4):402-8).

### Statistical analysis

Mann-Whitney U-test was used to asses statistically significant differences in the expression levels of the circulating EMT-TF mRNAs between cancer patients and controls. For the EMT-TFs with a significantly higher levels in CRC and PC patients, sensitivity and specificity were estimated using the optimum cut-off points determined by ROC curves analysis. A p-value of <0.05 was deemed to be of statistical significance. All statistical analysis was conducted using StatDirect software (StatsDirect Ltd, Altrincham CHESHIRE,UK).

### Patients and Methods

The pilot study included 31 patients with colorectal neoplasia. Of these, 26 patients had colorectal cancer, 4 patients had rectal cancer and underwent neo-adjuvant radio-chemo therapy prior to surgery (resulting in complete pathological remission, ypT0N0 stage, according to AJCC staging system), plus one patient with an advanced adenoma (showing high-grade dysplasia). Other 10 patients had pancreatic cancer, and 16 healthy subjects were used as controls. All samples were processed as above described after obtaining patient informed consent.

### Results

### 1. High levels of TWIST1 and SLUG mRNAs are present in blood cells of CRC patients

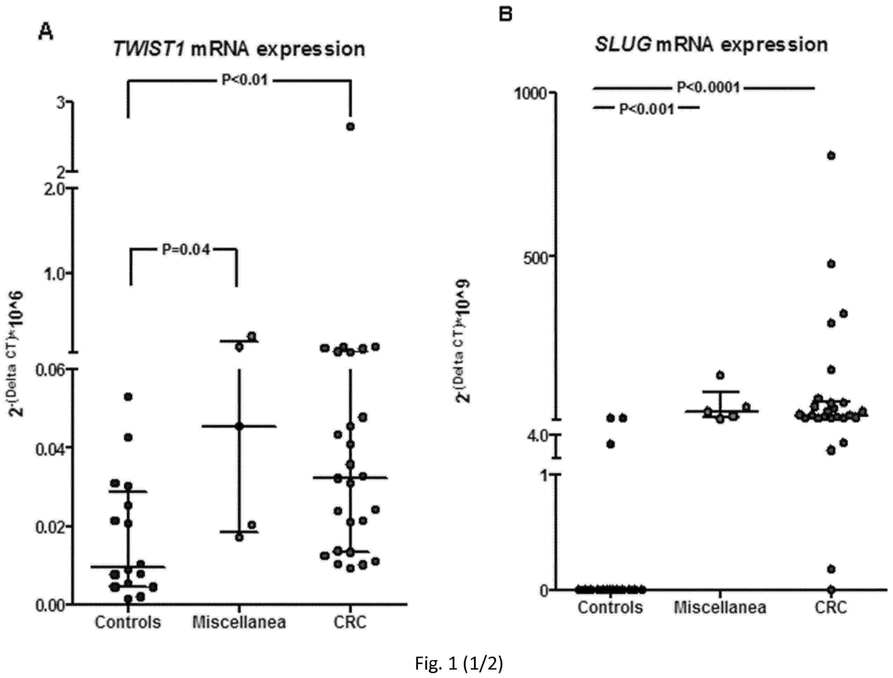

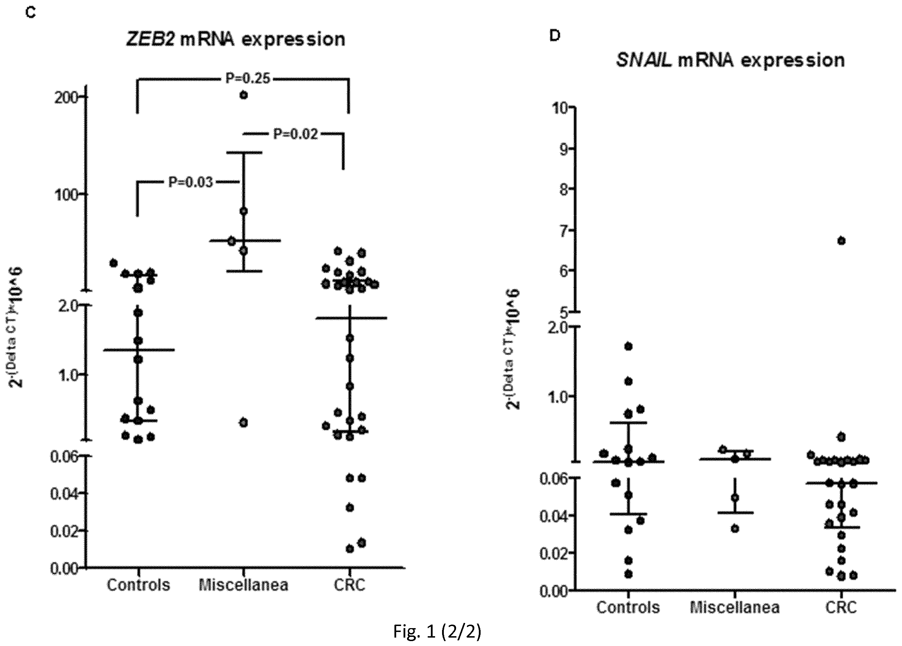

In 16 healthy controls *TWIST1*, *ZEB2* and *SNAIL* mRNAs were detected (16/16, 100%), while *SLUG* mRNA was detectable in 3 of 16 (18.75%) cases. In blood samples from 31 patients with a colorectal neoplasia, authors detected *TWIST1*, *ZEB2* and *SNAIL* in all (31/31, 100%) cases, and *SLUG* in 30 out of 31 (96.15%) cases. As Shown in Figure 1, CRC patients had higher levels of both *TWIST1* (Fig 1A, absolute quantification, median, controls 9.8E-9 vs CRC 3.22E-8; p<0.01) and *SLUG* (Fig. 1B, controls 9.09E-13 vs CRC, 1.76E-08; p<0.001), but not of *ZEB2* (Fig. 1C, controls 1.35E-06 vs CRC 7.45E-06; p=0.26) and of *SNAIL* (Fig. 1D, controls 7.61E-08 vs CRC 5.74E-08; p=0.20). Accordingly, the levels of *TWIST1* and *SLUG* mRNAs in blood cells of cancer patients were on average 3 and 4000 times higher, respectively, than in controls (relative quantification).

The study also included a set of blood samples (Miscellanea) from 4 patients with rectal cancer who underwent neo-adjuvant radio-chemo therapy prior to surgery, resulting in complete histological remission from the disease (ypT0N0), and from one patient with an advanced adenoma (showing high-grade dysplasia). In the Miscellanea set authors detected a significantly higher expression of *TWIST1*, *SLUG*, and *ZEB2* mRNA than in healthy controls. However, in this set levels of *ZEB2* were also significantly higher than in cancer patients (5.13E-05 vs 7.45E-06 CRC patients, p=0.01, Figure 1C), thus likely providing a method to discriminate between patients undergoing remission and those prone to progression following neo-adjuvant radio-chemotherapy.

### Threshold values of TWIST1 and SLUG in blood cells identifying CRC patients.

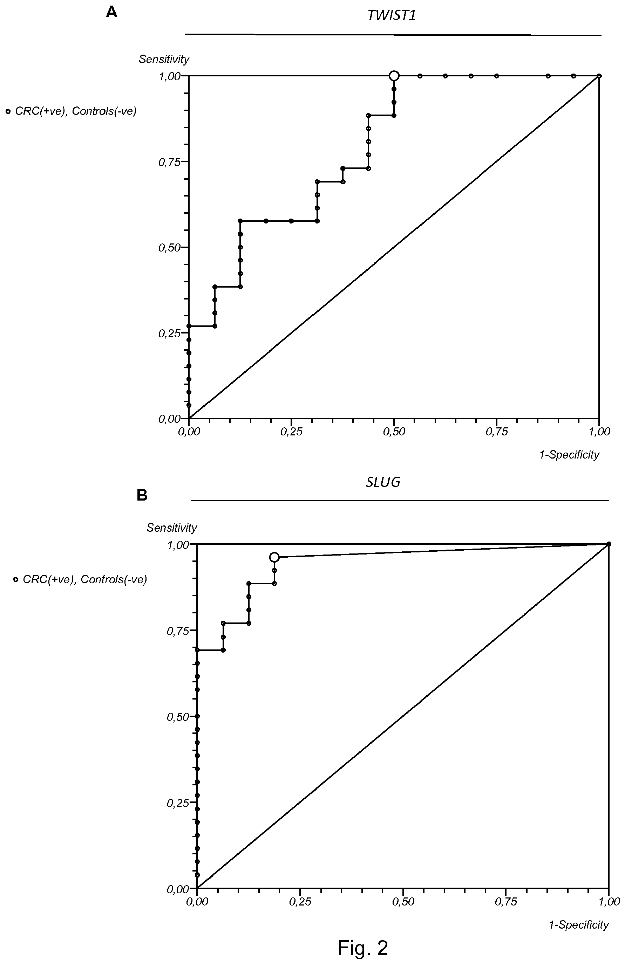

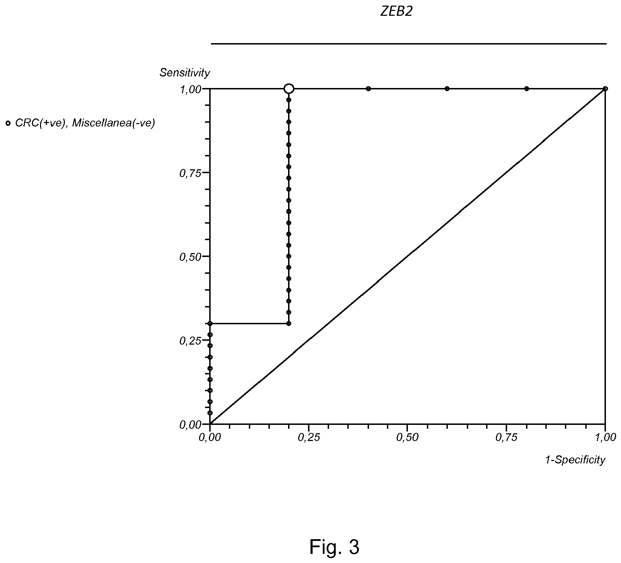

Authors next determined the optimum cut-off point for high levels of transcripts discriminating CRC patients by ROC curve analysis (Fig. 2). By this approach, high levels of *TWIST1* (above the cut-off, 9.30E-09) had 100% sensitivity and 50% specificity (Fig. 2A), and *SLUG* (above the cut-off, 1.83E-10) 96% sensitivity and 81% specificity in discriminating cancer patients from healthy controls (Fig. 2B). Furthermore, in the Miscellanea set, *ZEB2* mRNA levels above the optimum cut-off value of 4.08E-05 allowed discriminating before surgery between patients with CRC and those with rectal cancer who had complete histological response after neo-adjuvant therapy (sensitivity of 96% and specificity of 100%; Figure 3 and Table 2).

### Combined high levels of TWIST1 and SLUG mRNA enhance specificity in identifying cancer patients.

Discriminating CRC patients from healthy individuals by combining both *TWIST1* and *SLUG* high values (as determined by ROC curves), sensitivity was unaffected (96%), as all but one patients had high levels of both transcripts, while specificity was increased up to 93.3%, as high levels of both transcripts were present only in one control (Table 1).

**Table 1. Sensitivity and specificity for colorectal cancer of circulating levels of TWIST1 and SLUG mRNAs above the optimum cut-off values determined by ROC curve analysis.**

| | EMT-TFs | | |
|---|---|---|---|
| | *TWIST1* | *SLUG* | *Combined** |
| CRC Patients | | | |
| TP | 26 | 25 | 25 |
| FN | 0 | 1 | 1 |
| | | | |
| *Sensitivity* | *100%* | *96%* | *96%* |
| | | | |
| Controls | | | |
| TN | 8 | 13 | 15 |
| FP | 8 | 3 | 1 |
| *Specificity* | *50%* | *81%* | *93.3%* |

| | | | |
|---|---|---|---|
| *Patients presenting simultaneous levels above the optimum cut-off values of both *TWIST1* and *SLUG.* TP= True Positive FN = False Negative | | | |

**Table 2. Sensitivity and specificity of ZEB2 circulating mRNA levels* for discriminating patients with colo-rectal cancer from those with rectal cancer who underwent complete pathological remission following neo-adjuvant chemotherapy and radiotherapy.**

| | EMT-TF |
|---|---|
| | *ZEB2* |
| CRC Patients | |
| TP | 25 |
| FP | 1 |
| *Sensitivity* | *96*% |
| Rectal cancer, complete remission* | |
| TN | 4 |
| FN | 0 |
| *Specificity* | *100*% |

| | |
|---|---|
| *Patients with rectal cancer treated with neo-adjuvant therapy and complete histological response. TP= True Positive FN = False Negative | |

### 2. High levels of TWIST1, SLUG, and ZEB2 mRNAs are present in blood cells of pancreatic cancer (PC) patients.

**The levels of EMT-TF mRNA in the blood differ between PC patients and healthy individuals.**

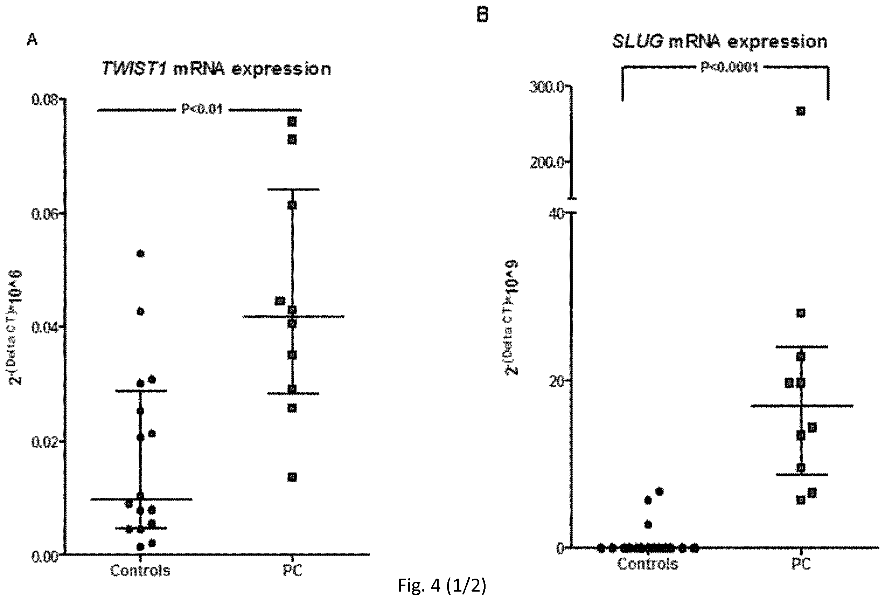

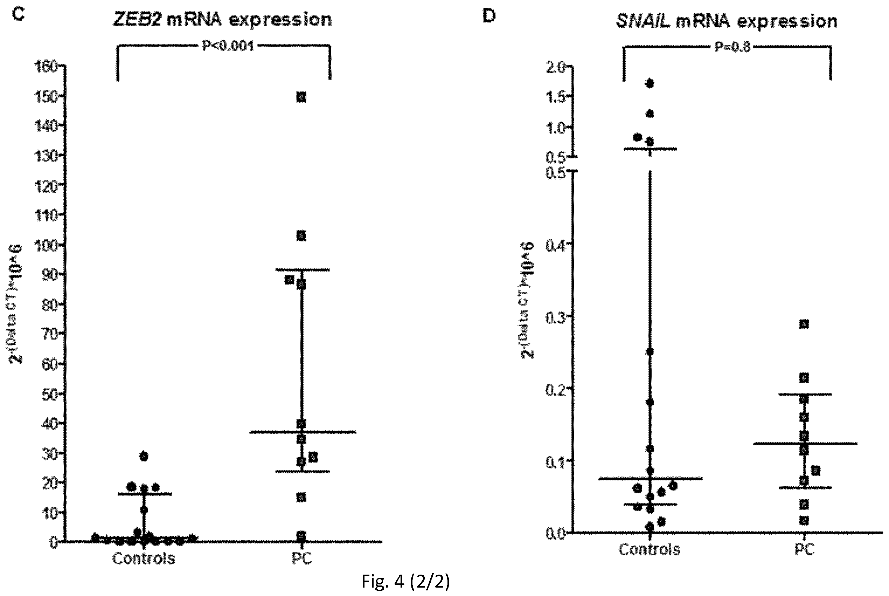

As Shown in Figure 4, compared to healthy controls, 10 PC patients had higher levels of *TWIST1* (Fig. 4A, absolute quantification, median, controls 9.8E-9 vs PC 4.10E-8; p<0.001) and of *SLUG* (Fig. 4B, controls 9.09E-13 vs PC, 17.00E-08; p<0.0001), as well as of *ZEB2* (Fig. 4C, controls 1.35E-06 vs PC 36.96E-06; p<0.001), but not of *SNAIL* (Fig. 4D, controls 7.61E-08 vs PC 12.00E-08; p=0.12). Accordingly, the circulating levels of *TWIST1*, *SLUG*, and *ZEB2* mRNAs in blood of PC patients were on average 4, 4000, and 22 times higher, respectively, than in controls (relative quantification).

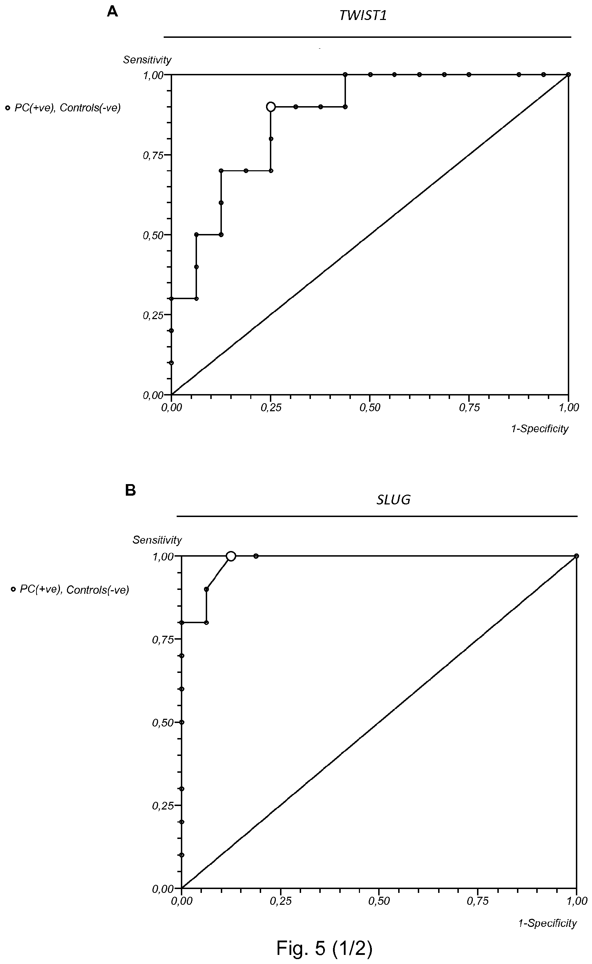

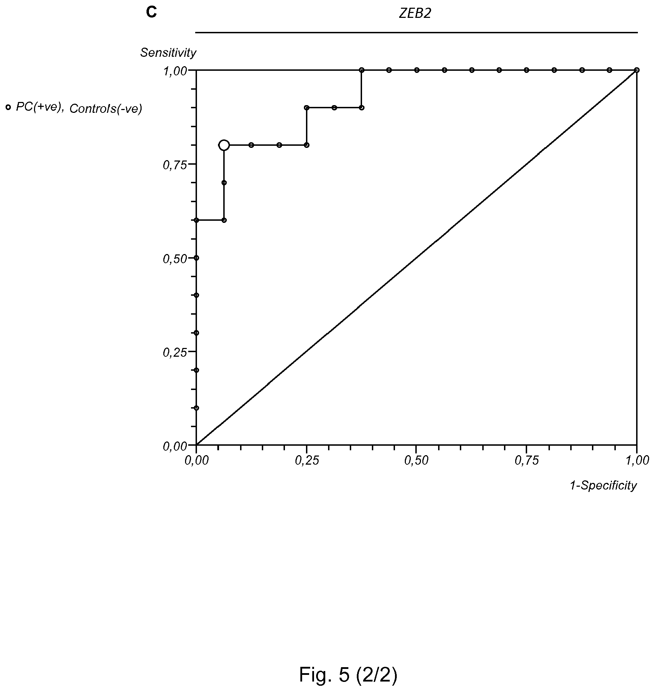

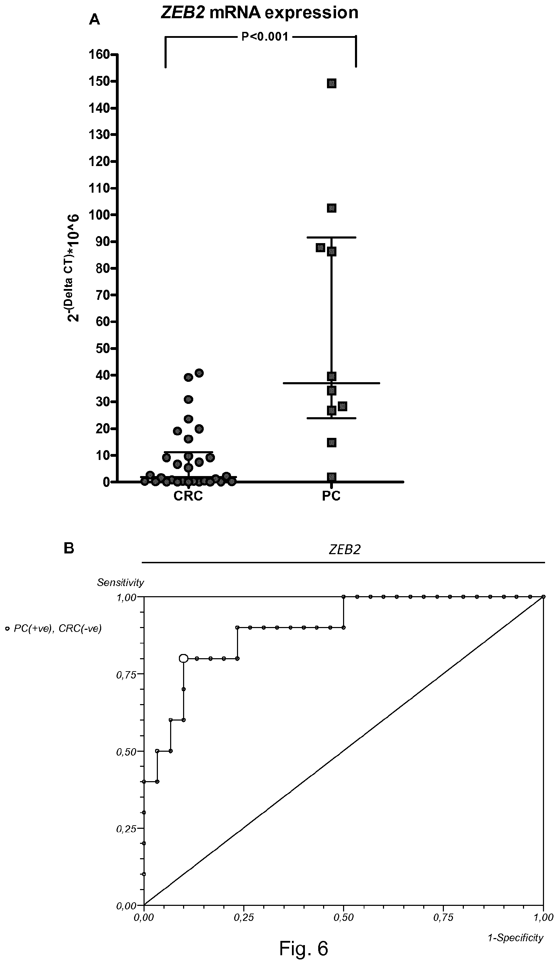

**Threshold values of EMT-TFs in blood identifying PC patients.** By ROC curve analysis, high levels of *TWIST1* (above the cut-off, 2.57E-8) had 90% sensitivity and 75% specificity (Fig. 5A), and *SLUG* (above the cut-off, 5.70E-9) 100% sensitivity and 87.5% specificity in discriminating cancer patients from healthy controls (Fig. 5B and Table 3). In addition, high levels of *ZEB2* (above the cut-off, 26.84E-6) discriminated between patients with PC and controls with 80%sensitivity and 93% specificity (Fig. 5c and Table 3). **ZEB2 mRNA levels are higher in blood cells of PC patients than in those of CRC patients.** In addition, comparing EMT-TF levels in blood between PC and CRC patients, we found that the levels of ZEB2 were significantly higher (p<0.001) in PC than in CRC patients. By relative quantification, the difference in transcript level was 8 times (Fig. 6A). Only patients with rectal cancer in remission after neo-adjuvant therapy (Miscellanea group) had levels of ZEB2 mRNA similar to those of patients with PC.

**Concomitant high levels of *TWIST1, SLUG,* and *ZEB2* mRNA enhance specificity in identifying PC patients, and discriminate PC from CRC patients.** Discriminating PC patients from healthy individuals by combining *TWIST1, SLUG* and *ZEB2* high-values (as determined by ROC curves), sensitivity was 80%, as all but two patients had high levels of the three transcripts, while specificity was increased up to 100%, as concomitant high-levels of these transcripts were never detected in controls (Table 3). Furthermore, the specificity of the *TWIST1*-*SLUG*-*ZEB2* high-level signature in discriminating patients with PC from those with CRC was 88%, as only 3 CRC patients had ZEB2 levels above the cut-off value for PC (Figure 6B).

**Table 3. Sensitivity and specificity for pancreatic cancer of circulating levels of TWIST1, SLUG, and ZEB2 mRNAs above the optimum cut-off values determined by ROC curve analysis.**

| | EMT-TFs | | | |
|---|---|---|---|---|
| | *TWIST1* | *SLUG* | *ZEB2* | *Concomitant** |
| PC Patients | | | | |
| TP | 9 | 10 | 8 | 8 |
| FN | 1 | - | 2 | 2 |
| | | | | |
| *Sensitivity* | *90%* | *100%* | *80%* | *80%* |
| | | | | |
| Controls | | | | |
| TN | 12 | 14 | 15 | 16 |
| FP | 4 | 2 | 1 | - |
| *Specificity* | *75%* | *87.5%* | *93.7%* | *100%* |

| | | | | |
|---|---|---|---|---|
| *Patients presenting simultaneous levels above the optimum cut-off values of *TWIST1*, *SLUG* and *ZEB2.* TP= True Positive FN = False Negative. | | | | |

## Claims

1. A method for determining if a subject is affected by a cancer comprising the step of assaying a biological sample from said subject for the presence of a panel of mRNAs encoding for transcription factors involved in epithelial to mesenchymal transition, wherein :
a) said panel comprises at least TWIST1, SLUG and ZEB2 mRNAs,
b) the increase of mRNA levels of both TWIST1 and SLUG genes but not of ZEB2 gene with respect to control samples is indicative of a colorectal cancer,
c) the increase of mRNA levels of all of TWIST1, SLUG and ZEB2 genes with respect to control samples is indicative of a pancreatic cancer,
wherein said biological sample is a blood cell sample.

2. The method for determining if a subject is affected by a cancer according to claim 1 wherein the subject is not administered an antitumor treatment.

3. The method according to any one of claims 1-2 wherein said blood cell sample is a Circulating Tumor Cells (CTCs) enriched sample.

4. The method according to claim 3 wherein said Circulating Tumor Cells (CTCs) enriched sample is a Peripheral Blood Mononuclear Cell (PBMC) sample.

5. The method according to any of previous claims wherein relevant cut-offs are as follows :
- TWIST1 mRNA : 9.30E-09 if the subject is affected by a colorectal cancer, or 2.57E-8 if the subject is affected by a pancreatic cancer;
- SLUG mRNA: 1.83E-10 if the subject is affected by a colorectal cancer, or 5.70E-9 if the subject is affected by a pancreatic cancer;
- ZEB2 : 4.08E-05 if the subject is affected by a colorectal cancer, as indication upon cancer treatment of partial or full remission of colorectal cancer, and/or colorectal cancer stage variation and/or treatment responsiveness, or 26.84E-6 if the subject is affected by pancreatic cancer.

6. The method according to any of previous claims wherein the levels of mRNAs are obtained by RT-PCR.

7. Use of a quantitative RT-PCR kit for working the method according to any of previous claims including:
- retrotranscribing means to get specific cDNAs;
- specific amplification probes for amplifying specific cDNAs;
- detecting means to detect and measure the level of amplified specific cDNAs.

8. Use of the quantitative RT-PCR kit according to claim 7 wherein said amplification probes are able to amplify:
- the nt. 781-835 region of *TWIST1,* Acc. No.: NM_000474.3, (SEQ ID No. 1);
- the nt. 711-819 region of *SLUG,* Acc. No.: NM_003068.3, (SEQ ID No. 2);
- the nt. 1262-1352 region of *ZEB2* Acc. No.: NM_014795.3, (SEQ ID No. 3);
- the nt. 174-244 region of *SNAIL,* Acc. No.: NM_005985.3, (SEQ ID No. 4).

9. Use of the quantitative RT-PCR kit according to claim 7 or 8 wherein said amplification probes have essentially the sequences:
TWIST1-Forward AGCAAGATTCAGACCCTCAAGCT (SEQ ID No. 8);
TWIST1-Reverse CCTGGTAGAGGAAGTCGATGTACCT (SEQ ID No. 9);
SLUG-Forward TGTTTGCAAGATCTGCGGC (SEQ ID No. 10);
SLUG-Reverse TGCAGTCAGGGCAAGAAAAA (SEQ ID No. 11);
ZEB2-Forward GCTACACGTTTGCCTACCGC (SEQ ID No. 12);
ZEB2-Reverse CGATTACCTGCTCCTTGGGTT (SEQ ID No. 13).

## Patentansprüche

1. Verfahren zur Bestimmung, ob ein Subjekt von einem Krebs betroffen ist, umfassend den Schritt des Testens einer biologischen Probe von dem Subjekt auf das Vorhandensein eines Panels von mRNAs, die für Transkriptionsfaktoren kodieren, die Epithel-Mesenchmal-Übergang beteiligt sind, wobei:
a) die Platte mindestens TWIST1, SLUG und ZEB2 mRNAs umfasst,
b) die Zunahme der mRNA-Spiegel sowohl der TWIST1-als auch der SLUG-Gene, aber nicht des ZEB2-Gens in Bezug auf Kontrollproben ein Indikator für einen Darmkrebs ist,
c) die Zunahme der mRNA-Spiegel aller TWIST1-, SLUG- und ZEB2-Gene in Bezug auf Kontrollproben ein Hinweis auf einen Bauchspeicheldrüsenkrebs ist,
wobei die biologische Probe eine Blutzellenprobe ist.

2. Verfahren zur Bestimmung, ob ein Subjekt von einem Krebs nach Anspruch 1 betroffen ist, wobei dem Subjekt keine Antitumorbehandlung verabreicht wird.

3. Verfahren nach einem der Ansprüche 1 bis 2, wobei die Blutzellprobe eine mit zirkulierenden Tumorzellen (Circulating Tumor Cells, CTCs) angereicherte Probe ist.

4. Verfahren nach Anspruch 3, wobei die mit zirkulierenden Tumorzellen (CTCs) angereicherte Probe eine Probe aus periphären mononukleären Blutzellen (Peripheral Blood Mononuclear Cells, PBMC) ist.

5. Verfahren nach einem der vorhergehenden Ansprüche, wobei relevante Abgrenzungen wie folgt sind:
- TWIST1 mRNA: 9.30E-09, wenn das Subjekt von einem Darmkrebs betroffen ist, oder 2.57E-8, wenn das Subjekt von einem Bauchspeicheldrüsenkrebs betroffen ist;
- SLUG mRNA: 1.83E-10, wenn das Subjekt von einem Darmkrebs betroffen ist, oder 5.70E-9, wenn das Subjekt von einem Bauchspeicheldrüsenkrebs betroffen ist;
- ZEB2: 4.08E-05, wenn das Subjekt von einem Darmkrebs betroffen ist, als Hinweis auf die Krebsbehandlung der partiellen oder vollen Remission von Darmkrebs und/oder einer Variation des Darmkrebsstadiums und/oder des Ansprechens auf die Behandlung, oder 26.84E-6, wenn das Subjekt von Bauchspeicheldrüsenkrebs betroffen ist.

6. Verfahren nach einem der vorhergehenden Ansprüche, wobei die Mengen an mRNAs durch RT-PCR erhalten werden.

7. Verwendung eines quantitativer RT-PCR-Kits zur Durchführung des Verfahrens nach einem der vorangehenden Ansprüche, einschließlich:
- Retrotranskriptionsmittel, um spezifische cDNAs zu erhalten;
- spezifischer Amplifikationssonden zur Amplifikation spezifischer cDNAs;
- Erfassungsmittel zum Erfassen und Messen des Niveaus an amplifizierten spezifischen cDNAs.

8. Verwendung des quantitativen RT-PCR-Kits nach Anspruch 7, wobei die Verstärkungssonden in der Lage sind, die Folgenden verstärken:
- die nt. 781-835-Region TWIST1, Zugangsnr.: NM_000474.3, (SEQ ID Nr. 1);
- die nt. 711-819-Region von SLUG, Zugangsnr.: NM_003068.3, (SEQ ID Nr. 2);
- die nt. 1262-1352-Region von ZEB2 Zugangsnr.: NM_014795.3, (SEQ ID Nr. 3);
- die nt. 174-244-Region von SNAIL, Zugangsnr.: NM_005985.3, (SEQ ID Nr. 4).

9. Verwendung des quantitativen RT-PCR-Kits nach Anspruch 7 oder 8, wobei die Amplifikationssonden im Wesentlichen die Folgenden Sequenzen aufweisen:
TWISTI-Vorwärts AGCAAGATTCAGACCCTCAAGCT (SEQ ID Nr. 8) ;
TWIST1-Rückwärts CCTGGTAGAGGAAGTCGATGTACCT (SEQ ID Nr. 9) ;
SLUG-Vorwärts TGTTTGCAAGATCTGCGGC (SEQ ID Nr. 10);
SLUG-Rückwärts TGCAGTCAGGGCAAGAAAAA (SEQ ID Nr. 11);
ZEB2-Vorwärts GCTACACGTTTGCCTACCGC (SEQ ID Nr. 12);
ZEB2-Rückwärts CGATTACCTGCTCCTTGGGTT (SEQ ID Nr. 13).

## Revendications

1. Procédé permettant de déterminer si un sujet est atteint d'un cancer, comprenant l'étape d'analyse d'un échantillon biologique dudit sujet à la recherche de la présence d'un panel d'ARNm codant pour des facteurs de transcription impliqués dans la transition épithéliale à mésenchymateuse, dans lequel :
a) ledit panel comprend au moins les ARNm de TWIST1, SLUG et ZEB2,
b) l'augmentation des niveaux d'ARNm des gènes TWIST1 et SLUG mais pas du gène ZEB2 relativement aux échantillons témoins indique un cancer colorectal,
c) l'augmentation des niveaux d'ARNm de l'ensemble des gènes TWIST1, SLUG et ZEB2 relativement aux échantillons témoins indique un cancer pancréatique,
dans lequel ledit échantillon biologique est un échantillon de cellules sanguines.

2. Procédé permettant de déterminer si un sujet est atteint d'un cancer selon la revendication 1, dans lequel aucun traitement antitumoral n'est administré au sujet.

3. Procédé selon l'une quelconque des revendications 1 et 2, dans lequel ledit échantillon de cellules sanguines est un échantillon enrichi en cellules tumorales circulantes (CTC).

4. Procédé selon la revendication 3, dans lequel ledit échantillon enrichi en cellules tumorales circulantes (CTC) est un échantillon de cellules mononucléaires de sang périphérique (PBMC).

5. Procédé selon l'une quelconque des revendications précédentes, dans lequel des seuils pertinents sont les suivantes :
- ARNm de TWIST1 : 9,30E-09 si le sujet est atteint d'un cancer colorectal, ou 2,57E-8 si le sujet est atteint d'un cancer pancréatique ;
- ARNm de SLUG : 1,83E-10 si le sujet est atteint d'un cancer colorectal, ou 5,70E-9 si le sujet est atteint d'un cancer pancréatique ;
- ZEB2 : 4,08E-05 si le sujet est atteint d'un cancer colorectal, en tant qu'indication après traitement contre le cancer de rémission partielle ou totale d'un cancer colorectal, et/ou d'une modification du stade du cancer colorectal et/ou de la réponse au traitement, ou 26,84E-6 si le sujet est atteint d'un cancer pancréatique.

6. Procédé selon l'une quelconque des revendications précédentes, dans lequel les niveaux d'ARNm sont obtenus par RT-PCR.

7. Utilisation d'un kit de RT-PCR quantitative pour la mise en oeuvre du procédé selon l'une quelconque des revendications précédentes, comprenant :
- un moyen de rétrotranscription pour obtenir des ADNc spécifiques ;
- des sondes d'amplification spécifiques pour amplifier les ADNc spécifiques ;
- un moyen de détection pour détecter et mesurer le niveau d'ADNc spécifiques amplifiés.

8. Utilisation du kit de RT-PCR quantitative selon la revendication 7, dans lequel lesdites sondes d'amplification sont capables d'amplifier :
- la région nt 781 à 835 de *TWIST1*, n° d'accès : NM_000474.3, (SEQ ID NO : 1) ;
- la région nt 711 à 819 de *SLUG*, n° d'accès : NM_003068.3, (SEQ ID NO : 2) ;
- la région nt 1262 à 1352 de *ZEB2*, n° d'accès : NM_014795.3, (SEQ ID NO : 3) ;
- la région nt 174 à 244 de *SNAIL*, n° d'accès : NM_005985.3, (SEQ ID NO : 4).

9. Utilisation du kit de RT-PCR quantitative selon la revendication 7 ou 8, dans lequel lesdites sondes d'amplification ont sensiblement les séquences :
TWIST1-sens AGCAAGATTCAGACCCTCAAGCT (SEQ ID NO : 8) ;
TWIST1-antisens CCTGGTAGAGGAAGTCGATGTACCT (SEQ ID NO : 9) ;
SLUG-sens TGTTTGCAAGATCTGCGGC (SEQ ID NO : 10) ;
SLUG-antisens TGCAGTCAGGGCAAGAAAAA (SEQ ID NO : 11) ;
ZEB2-sens GCTACACGTTTGCCTACCGC (SEQ ID NO : 12) ;
ZEB2-antisens CGATTACCTGCTCCTTGGGTT (SEQ ID NO : 13).


### SEQUENCE LISTING

<110> Humanitas Mirasole S.p.A.
<120> High levels of EMT-TFs for the diagnosis of cancer, in particular of colorectal (CRC) and pancreatic (PC) cancer
<130> BE 121564
<160> 15
<170> PatentIn version 3.5
<210> 1
   <211> 1669
   <212> DNA
   <213> Homo sapiens
<400> 1
   gaggtataag agcctccaag tctgcagctc tcgcccaact cccagacacc tcgcgggctc 60
   tgcagcaccg gcaccgtttc caggaggcct ggcggggtgt gcgtccagcc gttgggcgct 120
   ttctttttgg acctcggggc catccacacc gtcccctccc cctcccgcct ccctccccgc 180
   ctcccccgcg cgccctcccc gcggaggtcc ctcccgtccg tcctcctgct ctctcctccg 240
   cgggccgcat cgcccgggcc ggcgccgcgc gcgggggaag ctggcgggct gaggcgcccc 300
   gctcttctcc tctgccccgg gcccgcgagg ccacgcgtcg ccgctcgaga gatgatgcag 360
   gacgtgtcca gctcgccagt ctcgccggcc gacgacagcc tgagcaacag cgaggaagag 420
   ccagaccggc agcagccgcc gagcggcaag cgcgggggac gcaagcggcg cagcagcagg 480
   cgcagcgcgg gcggcggcgc ggggcccggc ggagccgcgg gtgggggcgt cggaggcggc 540
   gacgagccgg gcagcccggc ccagggcaag cgcggcaaga agtctgcggg ctgtggcggc 600
   ggcggcggcg cgggcggcgg cggcggcagc agcagcggcg gcgggagtcc gcagtcttac 660
   gaggagctgc agacgcagcg ggtcatggcc aacgtgcggg agcgccagcg cacccagtcg 720
   ctgaacgagg cgttcgccgc gctgcggaag atcatcccca cgctgccctc ggacaagctg 780
   agcaagattc agaccctcaa gctggcggcc aggtacatcg acttcctcta ccaggtcctc 840
   cagagcgacg agctggactc caagatggca agctgcagct atgtggctca cgagcggctc 900
   agctacgcct tctcggtctg gaggatggag ggggcctggt ccatgtccgc gtcccactag 960
   caggcggagc cccccacccc ctcagcaggg ccggagacct agatgtcatt gtttccagag 1020
   aaggagaaaa tggacagtct agagactctg gagctggata actaaaaata aaaatatatg 1080
   ccaaagattt tcttggaaat tagaagagca aaatccaaat tcaaagaaac agggcgtggg 1140
   gcgcactttt aaaagagaaa gcgagacagg cccgtggaca gtgattccca gacgggcagc 1200
   ggcaccatcc tcacacctct gcattctgat agaagtctga acagttgttt gtgttttttt 1260
   tttttttttt tttgacgaag aatgttttta tttttatttt tttcatgcat gcattctcaa 1320
   gaggtcgtgc caatcagcca ctgaaaggaa aggcatcact atggactttc tctattttaa 1380
   aatggtaaca atcagaggaa ctataagaac acctttagaa ataaaaatac tgggatcaaa 1440
   ctggcctgca aaaccatagt cagttaattc tttttttcat ccttcctctg aggggaaaaa 1500
   caaaaaaaaa cttaaaatac aaaaaacaac attctattta tttattgagg acccatggta 1560
   aaatgcaaat agatccggtg tctaaatgca ttcatatttt tatgattgtt ttgtaaatat 1620
   ctttgtatat ttttctgcaa taaataaata taaaaaattt agagaaaaa 1669
<210> 2
   <211> 2101
   <212> DNA
   <213> Homo sapiens
<400> 2
   agttcgtaaa ggagccgggt gacttcagag gcgccggccc gtccgtctgc cgcacctgag      60
   cacggcccct gcccgagcct ggcccgccgc gatgctgtag ggaccgccgt gtcctcccgc     120
   cggaccgtta tccgcgccgg gcgcccgcca gacccgctgg caagatgccg cgctccttcc     180
   tggtcaagaa gcatttcaac gcctccaaaa agccaaacta cagcgaactg gacacacata     240
   cagtgattat ttccccgtat ctctatgaga gttactccat gcctgtcata ccacaaccag     300
   agatcctcag ctcaggagca tacagcccca tcactgtgtg gactaccgct gctccattcc     360
   acgcccagct acccaatggc ctctctcctc tttccggata ctcctcatct ttggggcgag     420
   tgagtccccc tcctccatct gacacctcct ccaaggacca cagtggctca gaaagcccca     480
   ttagtgatga agaggaaaga ctacagtcca agctttcaga cccccatgcc attgaagctg     540
   aaaagtttca gtgcaattta tgcaataaga cctattcaac tttttctggg ctggccaaac     600
   ataagcagct gcactgcgat gcccagtcta gaaaatcttt cagctgtaaa tactgtgaca     660
   aggaatatgt gagcctgggc gccctgaaga tgcatattcg gacccacaca ttaccttgtg     720
   tttgcaagat ctgcggcaag gcgttttcca gaccctggtt gcttcaagga cacattagaa     780
   ctcacacggg ggagaagcct ttttcttgcc ctcactgcaa cagagcattt gcagacaggt      840
   caaatctgag ggctcatctg cagacccatt ctgatgtaaa gaaataccag tgcaaaaact     900
   gctccaaaac cttctccaga atgtctctcc tgcacaaaca tgaggaatct ggctgctgtg     960
   tagcacactg agtgacgcaa tcaatgttta ctcgaacaga atgcatttct tcactccgaa    1020
   gccaaatgac aaataaagtc caaaggcatt ttctcctgtg ctgaccaacc aaataatatg    1080
   tatagacaca cacacatatg cacacacaca cacacacacc cacagagaga gagctgcaag    1140
   agcatggaat tcatgtgttt aaagataatc ctttccatgt gaagtttaaa attactatat    1200
   atttgctgat ggctagattg agagaataaa agacagtaac ctttctcttc aaagataaaa    1260
   tgaaaagcac attgcatctt ttcttcctaa aaaaatgcaa agatttacat tgctgccaaa    1320
   tcatttcaac tgaaaagaac agtattgctt tgtaatagag tctgtaatag gatttcccat     1380
   aggaagagat ctgccagacg cgaactcagg tgccttaaaa agtattccaa gtttactcca     1440
   ttacatgtcg gttgtctggt tgccattgtt gaactaaagc ctttttttga ttacctgtag      1500
   tgctttaaag tatattttta aaagggagga aaaaaataac aagaacaaaa cacaggagaa     1560
   tgtattaaaa gtatttttgt tttgttttgt ttttgccaat taacagtatg tgccttgggg     1620
   gaggagggaa agattagctt tgaacattcc tggcgcatgc tccattgtct tactatttta     1680
   aaacatttta ataatttttg aaaattaatt aaagatggga ataagtgcaa aagaggattc     1740
   ttacaaattc attaatgtac ttaaactatt tcaaatgcat accacaaatg caataataca     1800
   ataccccttc caagtgcctt tttaaattgt atagttgatg agtcaatgta aatttgtgtt     1860
   tatttttata tgattgaatg agttctgtat gaaactgaga tgttgtctat agctatgtct     1920
   ataaacaacc tgaagacttg tgaaatcaat gtttcttttt taaaaaacaa ttttcaagtt     1980
   ttttttacaa taaacagttt tgatttaaaa tctcgtttgt atactatttt cagagacttt     2040
   acttgcttca tgattagtac caaaccactg tacaaagaat tgtttgttaa caagaaaaaa     2100
   a                                                                     2101
<210> 3
   <211> 9243
   <212> DNA
   <213> Homo sapiens
<400> 3
   atttcatttc ttccactaaa gcgtttgcgg agacttcaag gtataatcta tcccagatcc        60
   tttcccagag agaaacttgg cgatcacgtt ttcacatgat gctcacgctc agggcgcttc       120
   aattatccct ccccacaaag ataggtggcg cgtgtttcag ggtctctcgt ctctctccta       180
   cagaaaagaa aaagaaaaaa atgtcattag aagaggcgta acacgtcagt ccgtccccag       240
   gtttgtgttt cctggagtgg ccgaaagaga tcagttctaa cctgctctgc aggaataacg       300
   gtcctgcctc ccgacactct tggcgaggtt tttgtacagt ttgctccggg agctgtttct       360
   tcgcttccac ctttttctcc cccacacttc gcggcttctt catgcttttt cttctcacca       420
   tttctggcca aaactacaaa caagacttcg cagatcgagc ctgcgtgctg ccgaagcagg       480
   gcgccgagtc catgcgaact gccatctgat ccgctcttat caatgaagca gccgatcatg       540
   gcggatggcc cccggtgcaa gaggcgcaaa caagccaatc ccaggaggaa aaacgtggtg       600
   aactatgaca atgtagtgga cacaggttct gaaacagatg aggaagacaa gcttcatatt       660
   gctgaggatg acggtattgc caaccctctg gaccaggaga cgagtccagc tagtgtgccc       720
   aaccatgagt cctccccaca cgtgagccaa gctctgttgc caagagagga agaggaagat       780
   gaaataaggg agggtggagt ggaacacccc tggcacaaca acgagattct acaagcctct       840
   gtagatggtc cagaagaaat gaaggaagac tatgacacta tggggccaga agccacgatc      900
   cagaccgcaa ttaacaatgg tacagtgaag aatgcaaatt gcacatcaga ttttgaggaa      960
   tactttgcca aaagaaaact ggaggaacgc gatggtcatg cagtcagcat cgaggagtac     1020
   cttcagcgca gtgacacagc cattatttac ccagaagccc ctgaggagct gtctcgcctt     1080
   ggcacgccag aggccaatgg gcaagaagaa aatgacctgc cacctggaac tccagatgct     1140
   tttgcccaac tgctgacctg cccctactgc gaccggggct acaagcgctt gacatcactg     1200
   aaggagcaca tcaagtaccg ccacgagaag aatgaagaga acttttcctg ccctctctgt     1260
   agctacacgt ttgcctaccg cacccagctc gagcggcata tggtgacaca caagccaggg     1320
   acagatcagc accaaatgct aacccaagga gcaggtaatc gcaagttcaa atgcacagag     1380
   tgtggcaagg ccttcaaata taaacaccat ctgaaagaac acctgcgaat tcacagtggt     1440
   gaaaaacctt acgagtgccc aaactgcaag aaacgtttct cccattctgg ttcctacagt     1500
   tcgcacatca gcagcaagaa atgtattggt ttaatctctg taaatggccg aatgagaaac     1560
   aatatcaaga cgggttcttc ccctaattct gtttcttctt ctcctactaa ttcagccatt     1620
   acccagttaa gaaacaagtt ggagaatgga aaaccactta gtatgtctga acagacaggc     1680
   ttacttaaaa ttaaaacaga accactagac ttcaatgact ataaagttct tatggctaca     1740
   cacgggttta gtggcactag tccctttatg aatggtgggc ttggagccac cagcccttta     1800
   ggagttcatc catctgctca gagtccaatg cagcacttag gtgtagggat ggaagcccct     1860
   ttacttgggt ttcccaccat gaatagtaat ttaagtgagg tacaaaaggt tctacagatt     1920
   gtggacaata ctgtttccag gcaaaaaatg gactgcaagg ctgaagaaat ttcaaagttg     1980
   aaaggttatc acatgaagga tccatgctct caacctgagg aacaaggagt tacttctcct     2040
   aatattccgc ctgtcggtct tccggtagtg agtcataatg gtgccactaa aagtattatt     2100
   gactatacgt tggaaaaagt caatgaagcc aaagcttgcc tccagagctt gactactgac     2160
   tcaaggagac agatcagtaa tataaagaaa gagaagctac gtactttaat agatttggtc     2220
   actgatgaca aaatgattga gaaccacaac atatccactc cattttcatg ccagttctgt     2280
   aaagaaagtt ttcctggccc catccctttg catcagcatg aacgttacct ttgtaagatg     2340
   aatgaagaga tcaaggcggt cctgcagcct catgaaaaca tagtccccaa caaagccgga     2400
   gtttttgttg ataataaagc cctcctcttg tcatctgtac tttctgagaa aggaatgaca     2460
   agccccatca acccatacaa ggaccacatg tctgtactca aagcatacta tgctatgaac     2520
   atggagccca actccgatga actgctgaaa atttccattg ctgtgggcct tcctcaggaa     2580
   tttgtgaagg aatggtttga acaacgaaaa gtctaccagt actcaaattc caggtcccca     2640
   tccctggaaa gaagctccaa gccgttagct cccaacagta accctcccac aaaagactct     2700
   ttattaccca ggtctcctgt aaaacctatg gactccataa catcaccatc tatagcagaa     2760
   ctccacaaca gtgttacgaa ttgtgatcct cctctcaggc taacaaaacc ttcccatttt     2820
   accaatatta aaccagttga aaaattggac cactccagga gtaatactcc ttctccctta     2880
   aatctttcct ccacatcttc taaaaactcc cacagtagtt catacactcc aaacagcttc     2940
   tcttctgagg agctccaggc tgagccttta gacttgtcat taccaaaaca aatgaaagaa     3000
   cccaaaagta ttatagccac aaagaacaaa acaaaagcta gtagcatcag tttagatcat     3060
   aacagtgttt cttcctcatc tgaaaactca gatgagcctc tgaacttgac ttttatcaag     3120
   aaggaatttt caaattcaaa taatctggac aacaaaagca ctaacccagt gttcagcatg     3180
   aacccattta gtgccaaacc tttatacaca gctcttccac ctcaaagcgc atttccccct    3240
   gctactttca tgccaccagt ccagaccagt attcctgggc tacgaccata cccaggactg     3300
   gatcagatga gcttcctacc acatatggcc tacacctacc caactggagc agctactttt     3360
   gctgatatgc agcaaaggag aaagtaccag cggaaacaag gatttcaggg agaattgctt     3420
   gatggagcac aagactacat gtcaggccta gatgatatga cagactccga ctcctgtctg     3480
   tctcgcaaaa agatcaagaa gacagagagt ggcatgtatg catgtgactt atgtgacaag     3540
   acattccaga aaagcagttc ccttctgcga cataaatacg aacacacagg aaaaagacca     3600
   catcagtgtc agatttgtaa gaaagcgttt aaacacaagc accaccttat cgagcactca     3660
   aggcttcact cgggcgagaa gccctatcag tgtgataaat gtggcaagcg cttctcacac     3720
   tcgggctcgt actcgcagca catgaatcac aggtattcct actgcaagcg ggaggcggag     3780
   gagcgggaag cggcggagcg cgaggcgcgc gagaaagggc acttggaacc caccgagctg     3840
   ctgatgaacc gggcttactt gcagagcatt acccctcagg ggtactctga ctcggaggag     3900
   agggagagta tgccgaggga tggcgagagc gagaaggagc acgagaaaga aggcgaggat     3960
   ggctacggga agctgggcag acaggatggc gacgaggagt tcgaggagga agaggaagaa     4020
   agtgaaaata aaagtatgga tacggatccc gaaacgatac gagatgaaga agagactgga     4080
   gatcactcca tggacgatag ttcggaggat gggaaaatgg aaaccaaatc agaccacgag     4140
   gaagacaata tggaagatgg catgtaataa actactgcat tttaagcttc ctattttttt     4200
   ttccagtagt attgttacct gcttgaaaac actgctgtgt taagctgttc atgcacgtgc     4260
   ctgacgcttc caggaagctg tagagaggga cagaaggggc ggttcagcca agacagatgt     4320
   agacggagtt ggagctgggt attgttaaaa actgcattat gcaaaaattt tgtacagtgt     4380
   taaggcctaa aaactgtgtg gttcagagac taattcctgt gtttaatagc atttatactt     4440
   taagcacaac tagaaaattg taagaattgc actctactta tgtatcacta caaactttaa     4500
   aaaactatgt ctaatttata ttaatacatt ttaaaaaggt gcccgcacta ccatacatca     4560
   gtatttttat tattattatt gttattcctt tttaatttaa tgtgctcgca ctacaatgca     4620
   tcagtattat gattcctctg tactttcctt tcgctattca tcaatttccc attttttttt 4680
   tcagcttaag taaccacaca attttaggcc tcaatttttt tttttttctg tgaaggaact 4740
   tgaagtgatg catgtgtgaa tttaagatac cgaagtctta aagtgacctg gacgtgaagg 4800
   aaaaagtaag atgagaaata aagaaagcct ttgtaaggtg gttttaaaag ccttatatgc 4860
   aaacctttta atctgtgttt ctgcaagtgc catccttgta cagtgttaag agggtaacat 4920
   gggttacctt tgcaccagct tcagtgttaa gctcaccctg ttctttgaag cacccatgtc 4980
   agtattagaa gaataggcag cagttcctta gtttacatat gtttgtgcaa ttattttctg 5040
   tacttttttg ttcattaatt ttgtcagtat tacaccaaac tgtttttgca acaaaaaaat 5100
   tttttttgca ttcatttaat tttaggtcaa ataacatttt atttatgtgg ctcattttat 5160
   atttcctaat tttatttatt tcatactgta gtgtacagta ttatagttct tcaatatata 5220
   gatatatttt agtaaaaaag gaacatgacg ttgatcattt gggcaaattt tacgtaaaga 5280
   gaagagcatt tattgtgttt tggaacatta attgtgagat gggatttttc aattttatta 5340
   ttttattttt gtttttttcc aattactgga aattccaaat ttgggaactt ttgatacgat 5400
   cttgtgaaaa cactgtattt tcgactgaaa attccacttt cttcatcttg ttttttagct 5460
   aaaaagaggg actgttaaat acaatgtatg ataccatgac aaaaatcttt cctgaattgt 5520
   ctttgtaaaa gtattattga attttcaatt tgtaatttct tttgaaaatg accatgctcg 5580
   aataaaaatg tagccaaact aagaatgtag ttaatgagtt ctgtactttt agagagtttt 5640
   ccttcaatga ccattaacat gtaacatgct ttatgcttat aataatgcta attatgtttt 5700
   tttcatataa ttttagttta gcaataattt tgactggtac caataactgt tttttaaaat 5760
   tccataccta tgtacagcaa ttttacagct tttctcaact gatcctgatt ccagattgtg 5820
   tatttttatg tgaggttata ttattcaaat ttagtctatt tactttacag acatttctac 5880
   ttttgcatta cgagtattta gagattatgt gttaaaaatt cacttctctg tccaaggggt 5940
   ctttgtgatt tattcaaaaa aaagtctaat ttcaaaaaga cagctattat tcagtgttat 6000
   ttataatatg taaccttttt taaaggattg ggatagttta tctcactttt tgaaatgcag 6060
   acagtagttt accgtttatc tgaaactaga aggcgtgggt gggagaggaa aagctaaaag 6120
   caaatgctaa caaaaataac cgtgattttc taagacagtt tttcagtttt tacaagatga 6180
   ccctaatatt cagaatatga atgtattcgt aggttttaca taatgacttt tatcaagaaa 6240
   ctagattctg cttcttaaat ctaattgcca agtgaagaat aacagaaaaa acagattacc 6300
   ttatcaaatt tacagctctt gaatatacag aactataata tagtagctgt ccatgtattt 6360
   tttctacttt agaatcaaag aagaaaagca tcattttgct attaaatttg ctaaaatttt 6420
   gagtatgata tttccagttg gcaagaacaa catatttata tttattcctt agccataata 6480
   ccactttcct aaatttcaca aaagtcattc tttgcaactt gaaactcaat agaaagtgtg 6540
   tatgtgtgtg tgtgtatata tatatatata tacacacaca cacatacaca gaaaggatgt 6600
   aatgaagata cagtaatagt tgagcagacc tttttagaaa aacatgtttt tagctctatc 6660
   ttcaaacttt ctggcagagg gggtgggggg ggcaggggga ggagtggcat caaaatgcta 6720
   tgcctcctgt tatccacagc ctagagtttt tatatttgga aagtttagaa aattctatcc 6780
   tcgtttctcc ttctttgaat ggcacaaata aatacactac ataaattttt ctggtttgaa 6840
   aggctctagg cgataacttt attaattcaa cctgaaaata tcaagccatt aaattttgtc 6900
   cgggtagaat aaatccctgt ggcctctttt aaagcaatgt aggtctctgt tgcccatggg 6960
   gcatatctgt gtcccaatcc acaagagata ggaccaacaa acaatgaatg tgcaacctaa 7020
   ctctttctcc ttggaaagaa gaaagtgtgc acgaagtaga ggagggtggg cagaccctgc 7080
   cttgcccctc ctgttacccc cttctctgtc atttgttcct aactccattt cataggcagg 7140
   ctcagaatac ctgagtctga aaatatcagg ataacacttg tgaattgtga caatcactac 7200
   aatgtcccat atctgaggag ttttttttaa tgctatttat ccgctggaca cgattgcaca 7260
   ttagggctgc ataatcctct aactctaggg aaaaataaaa acttttgatt tgtcttaaga 7320
   ttcttctcca aggtcgcaaa caagaaattc ccctccacaa ccaagagatg tgcattttag 7380
   taacatcaga tgtgttcttc tgttttatca actacttact cttcccacac gcttagttct 7440
   aaatctaacc tttcccccct cgaatagggg gcaggggagg atgaggaaac actggaacaa 7500
   ctgaacaccc ctgcccattt tctccaagag ccttttgtat tctagcatat ctgtgcaatc 7560
   ttttcttttt tcttcacatg acactgtaag cttaggcctg aaataactgg gaagagagat 7620
   gcgtatcaga atttctccgc aagagctaaa caaaacatac atcttcctta gcatgaattg 7680
   gactgggggc ggagtgggag ggcttggagg aaaggggaaa gaagggacta tatttgaata 7740
   aatatgaata aatgtattag atacttttca caatcagata acttttaaaa aggtcatttt 7800
   ttatctttct aataatgtaa gccttaataa aagcaaatct tagtcacaaa tttgaggaga 7860
   ctgcccaata ataagtttac atgtatttga actgaaaaat tgttaaccat gcttttgctc 7920
   caagatgtgt gaggccattc aggggctgta gggccctgga tatacacaca aacaagtgtg 7980
   tgtatatctg gagccccaca cattgtaata aacacagctg catttatttg actatgtgat 8040
   cccatgtaca tgtaaaaaca ttcaaacaaa cacactcagc ggatttattt attgtgcaat 8100
   ggggcaatta ttcaaataaa catgctcaat gcaattattt gaatctcaca ttgcatgttc 8160
   atcaatcata gcactaaaaa aagaggggga aaaaacacca aagaattcac atggggaaaa 8220
   aatatatata tgaaaaccac cttattatag attttatagg gcagctgagg ttatggctcc 8280
   cttcttaact gtaactcaac tattctgtat tcaatgacat ttgtttctaa tgattaattg 8340
   gttcactcac ttgatcatat aatagcaaac tttataaacc tgtattgtgt agagatgtga 8400
   aatctctata tttcaagagc agaagagttc tttctagaca ccttacatca agggacactg     8460
   gtccaattat tatcgcttat ataagcactc ctataaattc tgaaaaattt tatacatgca     8520
   acaaaacatt cctacatttg aagacattaa gaaaaatcac aggtgactca tctgatcatt     8580
   ctatatatta ataaatatta tgacatatat gtgaacacat cacaaatcat attggtgtac     8640
   caagaggcaa tttatgcctc tcttaagtat gtactgacat aacctaatat actaaaatgg     8700
   gaaggggctt ttagtcactg aaatatgcat cgtgtaacaa agatgaagaa aatacatggc     8760
   ttgtgcccat cataaaaaaa gattcagact gaaggcttag ctttggtttt ttcaattaaa     8820
   ttgttaaact gtgcacagtg attttttttt agaacttgag acatttgtga tgttggctgt     8880
   ttaaatcttt gttaccttcg ctgtgaattg aaattgtaca tatttagtaa atcatgcaga     8940
   caaaacaaac tttttagaca atatttttat tggagagttt tcttttcctg tatccatgtt     9000
   aaaaaaaaaa aagacctcct ttcccaaaat aaaaatgtca atactaaatt taaagaagta     9060
   taaaggaatg attgcttcct ttagagcaaa atatttaaat aaacatggag ataattggca     9120
   acatgttctt tttgggctag taggctgtgt ccaatttttt gggtctgatg tttcagaggg     9180
   cctctgtttc agggttgaag atgatatatt aatctcggaa ttaaacaaat gctattaaat     9240
   aac                                                                   9243
<210> 4
   <211> 1722
   <212> DNA
   <213> Homo sapiens
<400> 4
   attcattgcg ccgcggcacg gcctagcgag tggttcttct gcgctactgc tgcgcgaatc        60
   ggcgacccca gtgcctcgac cactatgccg cgctctttcc tcgtcaggaa gccctccgac       120
   cccaatcgga agcctaacta cagcgagctg caggactcta atccagagtt taccttccag       180
   cagccctacg accaggccca cctgctggca gccatcccac ctccggagat cctcaacccc       240
   accgcctcgc tgccaatgct catctgggac tctgtcctgg cgccccaagc ccagccaatt       300
   gcctgggcct cccttcggct ccaggagagt cccagggtgg cagagctgac ctccctgtca       360
   gatgaggaca gtgggaaagg ctcccagccc cccagcccac cctcaccggc tccttcgtcc       420
   ttctcctcta cttcagtctc ttccttggag gccgaggcct atgctgcctt cccaggcttg       480
   ggccaagtgc ccaagcagct ggcccagctc tctgaggcca aggatctcca ggctcgaaag       540
   gccttcaact gcaaatactg caacaaggaa tacctcagcc tgggtgccct caagatgcac       600
   atccgaagcc acacgctgcc ctgcgtctgc ggaacctgcg ggaaggcctt ctctaggccc       660
   tggctgctac aaggccatgt ccggacccac actggcgaga agcccttctc ctgtccccac       720
   tgcagccgtg ccttcgctga ccgctccaac ctgcgggccc acctccagac ccactcagat       780
   gtcaagaagt accagtgcca ggcgtgtgct cggaccttct cccgaatgtc cctgctccac       840
   aagcaccaag agtccggctg ctcaggatgt ccccgctgac cctcgaggct ccctcttcct       900
   ctccatacct gcccctgcct gacagccttc cccagctcca gcaggaagga ccccacatcc       960
   ttctcactgc catggaattc cctcctgagt gccccacttc tggccacatc agccccacag      1020
   gactttgatg aagaccattt tctggttctg tgtcctctgc ctgggctctg gaagaggcct      1080
   tcccatggcc atttctgtgg agggagggca gctggccccc agccctgggg gattcctgag      1140
   ctggcctgtc tgcgtgggtt tttgtatcca gagctgtttg gatacagctg ctttgagcta      1200
   caggacaaag gctgacagac tcactgggaa gctcccaccc cactcagggg accccactcc      1260
   cctcacacac acccccccac aaggaaccct caggccaccc tccacgaggt gtgactaact      1320
   atgcaataat ccacccccag gtgcagcccc agggcctgcg gaggcggtgg cagactagag      1380
   tctgagatgc cccgagccca ggcagctatt tcagcctcct gtttggtggg gtggcacctg      1440
   tttcccgggc aatttaacaa tgtctgaaaa gggactgtga gtaatggctg tcacttgtcg      1500
   ggggcccaag tggggtgctc tggtctgacc gatgtgtctc ccagaactat tctgggggcc      1560
   cgacaggtgg gcctgggagg aagatgttta catttttaaa ggtacactgg tatttatatt      1620
   tcaaacattt tgtatcaagg aaacgttttg tatagttata tgtacagttt attgatattc      1680
   aataaagcag ttaatttata tattaaaaaa aaaaaaaaaa aa                         1722
<210> 5
   <211> 1969
   <212> DNA
   <213> Homo sapiens
<220>
   <221> misc_feature
   <222> (57)..(61)
   <223> n is a, c, g, or t
<400> 5
   ccgtccgtcc gtcgtcctcc tcgcttgcgg ggcgccgggc ccgtcctcga gcccccnnnn        60
   nccgtccggc cgcgtcgggg cctcgccgcg ctctacctac ctacctggtt gatcctgcca       120
   gtagcatatg cttgtctcaa agattaagcc atgcatgtct aagtacgcac ggccggtaca       180
   gtgaaactgc gaatggctca ttaaatcagt tatggttcct ttggtcgctc gctcctctcc       240
   tacttggata actgtggtaa ttctagagct aatacatgcc gacgggcgct gacccccttc       300
   gcggggggga tgcgtgcatt tatcagatca aaaccaaccc ggtcagcccc tctccggccc       360
   cggccggggg gcgggccgcg gcggctttgg tgactctaga taacctcggg ccgatcgcac       420
   gccccccgtg gcggcgacga cccattcgaa cgtctgccct atcaactttc gatggtagtc       480
   gccgtgccta ccatggtgac cacgggtgac ggggaatcag ggttcgattc cggagaggga       540
   gcctgagaaa cggctaccac atccaaggaa ggcagcaggc gcgcaaatta cccactcccg      600
   acccggggag gtagtgacga aaaataacaa tacaggactc tttcgaggcc ctgtaattgg      660
   aatgagtcca ctttaaatcc tttaacgagg atccattgga gggcaagtct ggtgccagca      720
   gccgcggtaa ttccagctcc aatagcgtat attaaagttg ctgcagttaa aaagctcgta      780
   gttggatctt gggagcgggc gggcggtccg ccgcgaggcg agccaccgcc cgtccccgcc      840
   ccttgcctct cggcgccccc tcgatgctct tagctgagtg tcccgcgggg cccgaagcgt      900
   ttactttgaa aaaattagag tgttcaaagc aggcccgagc cgcctggata ccgcagctag      960
   gaataatgga ataggaccgc ggttctattt tgttggtttt cggaactgag gccatgatta     1020
   agagggacgg ccgggggcat tcgtattgcg ccgctagagg tgaaattctt ggaccggcgc     1080
   aagacggacc agagcgaaag catttgccaa gaatgttttc attaatcaag aacgaaagtc     1140
   ggaggttcga agacgatcag ataccgtcgt agttccgacc ataaacgatg ccgaccggcg     1200
   atgcggcggc gttattccca tgacccgccg ggcagcttcc gggaaaccaa agtctttggg     1260
   ttccgggggg agtatggttg caaagctgaa acttaaagga attgacggaa gggcaccacc     1320
   aggagtggag cctgcggctt aatttgactc aacacgggaa acctcacccg gcccggacac     1380
   ggacaggatt gacagattga tagctctttc tcgattccgt gggtggtggt gcatggccgt     1440
   tcttagttgg tggagcgatt tgtctggtta attccgataa cgaacgagac tctggcatgc     1500
   taactagtta cgcgaccccc gagcggtcgg cgtcccccaa cttcttagag ggacaagtgg     1560
   cgttcagcca cccgagattg agcaataaca ggtctgtgat gcccttagat gtccggggct     1620
   gcacgcgcgc tacactgact ggctcagcgt gtgcctaccc tacgccggca ggcgcgggta     1680
   acccgttgaa ccccattcgt gatggggatc ggggattgca attattcccc atgaacgagg     1740
   aattcccagt aagtgcgggt cataagcttg cgttgattaa gtccctgccc tttgtacaca     1800
   ccgcccgtcg ctactaccga ttggatggtt tagtgaggcc ctcggatcgg ccccgccggg     1860
   gtcggcccac ggcctggcgg agcgctgaga agacggtcga acttgactat ctagaggaag     1920
   taaaagtcgt aacaaggttt ccgtaggtga acctgcggaa ggatcatta                 1969
<210> 6
   <211> 21
   <212> DNA
   <213> Artificial Sequence
<220>
   <223> synthetic primer
<400> 6
   cgccgctaga ggtgaaattc t 21
<210> 7
   <211> 20
   <212> DNA
   <213> Artificial Sequence
<220>
   <223> synthetic primer
<400> 7
   ctttcgctct ggtccgtctt 20
<210> 8
   <211> 23
   <212> DNA
   <213> Artificial Sequence
<220>
   <223> synthetic primer
<400> 8
   agcaagattc agaccctcaa gct 23
<210> 9
   <211> 25
   <212> DNA
   <213> Artificial Sequence
<220>
   <223> synthetic primer
<400> 9
   cctggtagag gaagtcgatg tacct 25
<210> 10
   <211> 19
   <212> DNA
   <213> Artificial Sequence
<220>
   <223> synthetic primer
<400> 10
   tgtttgcaag atctgcggc 19
<210> 11
   <211> 20
   <212> DNA
   <213> Artificial Sequence
<220>
   <223> synthetic primer
<400> 11
   tgcagtcagg gcaagaaaaa 20
<210> 12
   <211> 20
   <212> DNA
   <213> Artificial Sequence
<220>
   <223> synthetic primer
<400> 12
   gctacacgtt tgcctaccgc 20
<210> 13
   <211> 21
   <212> DNA
   <213> Artificial Sequence
<220>
   <223> synthetic primer
<400> 13
   cgattacctg ctccttgggt t 21
<210> 14
   <211> 19
   <212> DNA
   <213> Artificial Sequence
<220>
   <223> synthetic primer
<400> 14
   cttccagcag ccctacgac 19
<210> 15
   <211> 18
   <212> DNA
   <213> Artificial Sequence
<220>
   <223> synthetic primer
<400> 15
   cggtggggtt gaggatct 18